Europäisches Patentamt

European Patent Office

Office européen des brevets

(19)

(11) Publication number: **0 041 650**
**A2**

(12)

# EUROPEAN PATENT APPLICATION

(21) Application number: **81104071.6**

(22) Date of filing: **27.05.81**

(51) Int. Cl.³: **C 12 N 1/08**
**C 12 N 1/00**

(30) Priority: **10.06.80 US 158181**

(43) Date of publication of application:
**16.12.81 Bulletin 81/50**

(84) Designated Contracting States:
**BE DE FR IT NL SE**

(71) Applicant: **Provesta Corporation**
**256 PLB, PRC**
**Bartlesville Oklahoma 74004(US)**

(72) Inventor: **Shay, Lucas Kingyuang**
**617 S.E.Castle Road**
**Bartlesville Oklahoma(US)**

(74) Representative: **Dost, Wolfgang, Dr. et al,**
**Patent- u. Rechtsanwälte Pagenberg-Dost-Altenburg**
**Galileiplatz 1**
**D-8000 München 80(DE)**

(54) A method of reducing the nucleic acid level in single cell protein and method for producing a single cell protein product.

(57) Single cell protein products are treated to reduce the level of nucleic acid content. Single cell protein products are also treated to produce single cell protein products of improved color, taste, and odor.

EP 0 041 650 A2

A method of reducing the nucleic acid level in single cell protein and method for producing a single cell protein product.

The invention relates to single cell protein having reduced nucleic acid content. In another aspect, the invention relates to a process for making such single cell protein.

### Background of the Invention

The term "single cell protein" has a variety of meanings. Commonly it is used to refer to a proteinaceous product isolated from single-celled microorganisms. Another common usage, employed herein unless otherwise indicated, applies the term to a whole cell product in which both protein and cell wall materials of single-celled microorganisms are present.

Rapid increases in world population have made the continued dependence on conventional food protein impractical. Single Cell Protein (SCP) is one possible solution to the world protein shortage. At present, SCP is mainly used for animal feed. However, in one form or another SCP is already utilized for human consumption and is expected to be of greater importance in the future.

SCP obtained by the cultivation of microorganisms generally contains a higher proportion of nucleic acids than conventional foods. Although the amount of nucleic acids present in SCP varies depending on the specific microorganism employed, generally about 5 to about 18 percent nucleic acids (dry weight) are present in SCP. The final metabolic product formed in humans from the purine moiety in nucleic acids is uric acid. Humans do not possess the enzyme uricase, which oxidizes uric acid to allantoin, a soluble and excretable metabolite. Consumption of a protein source high in nucleic acids results in a higher plasma level of uric acid. Since uric acid is only slightly soluble at physiological pH values, this can result in the formation of uric acid crystals in the joints, causing gout or gouty arthritis.

For human nutrition, the nucleic acid content of the SCP should be reduced to below about 9% if any substantial amount of SCP is used in the diet. The Recommended Daily Allowance for protein is 65 grams per day for a 70 kilogram adult male and the Protein Calorie Advisory Group of the United Nations System recommends that the amount of nucleic acid ingested per day from microbial protein should be less than 2 grams. Accordingly, the nucleic acid content of microbial protein should be less than about 3% by weight if these criteria are to be met when microbial protein is the only source of dietary protein. The nucleic acid content should be less than about 6% if the microbial protein constitutes 50% of the dietary protein. Where SCP contains cell wall materials in addition to microbial protein, the protein is typically present, for example, in yeasts in amounts of about 60% by weight of the total weight of SCP. Under these circumstances, the nucleic acid content should be less than about 2% by weight if the SCP is the sole source of dietary protein and less than about 4% by weight if the SCP constitutes 50% of dietary protein.

Numerous methods have been reported in the literature for the removal of nucleic acids from SCP. These methods include heat shock, acid treatment, base treatment, and enzymatic action. Some methods are relatively ineffective for nucleic acid removal or remove a large amount of the protein with the nucleic acids. Other methods are too complex or require such expensive steps that they are economically unattractive for large scale use. Still others cause the formation of non naturally occurring amino acids. One of these non naturally occurring amino acids, lysinoalanine, has been found, for example, to cause kidney lesions ("Protein Nutritional Quality of Foods and Feeds" Part 2, M. Friedman (Ed.), 1975, pp. 595-618).

In addition, the process employed for nucleic acid reduction can affect the purity of the SCP product as well as its flavor, odor, and color. For use as a dietary food supplement, for example, the SCP is desirably flavorless, odorless, and colorless to avoid affecting the natural color and flavor of foodstuffs with which it may be combined. Further, an SCP product substantially free of contaminating chemicals, such as, for example, ammonium hydroxide, is also desirable.

An object of my invention is a single cell protein product of reduced nucleic acid content and a process for producing same. Another object is such a process which does not result in substantially reduced protein content in the single cell protein product. Yet another object is such a process which does not result in the formation of substantial amounts of lysinoalanine. A further object is such a process which produces a single cell product having good color, taste, and odor characteristics and which is substantially free of contaminating chemicals. Further objects are single cell protein products having the characteristics achieved by my process. Other objects and advantages will be apparent to one skilled in the art from the disclosure and examples set out below.

## Summary of the Invention

Broadly my invention comprises producing a fluid containing cells by carrying out fermentation of at least one species of microorganism under aqueous fermentation conditions; treating said fluid containing cells with $NH_4OH$ at a pH in the range of about 7 to about 10 and at a temperature broadly in the range of about 65° to about 99°C, preferably from about 85°C to about 90°C; and separating said cells from said fluid. In a further aspect my invention comprises drying the thus separated cells to remove water and $NH_4OH$ thereby resulting in a dried nucleic acid reduced single cell protein product substantially free of contaminating chemicals such as ammonium hydroxide.

In another further aspect my invention comprises treating a fluid containing cells of at least one species of yeast at a pH in the range of about 8.5 to about 10.0.

In yet another aspect my invention comprises treating a fluid containing cells of at least one species of bacteria at a pH in the range of about 7 to about 9.0.

In yet other aspects my invention comprises nucleic acid reduced single cell protein products produced in accordance with my invention.

In yet further aspects, my invention comprises treatment of microbial cellular products with an oxidizing agent to produce a single cell protein of improved color, taste, and odor.

## I. Scope of Microorganisms

The process of the present invention is broadly applicable to bacteria and yeasts which are useful as a protein source. In view of the economic attractiveness, ready availability, and water miscibility of the lower straight chain alcohols as the feedstock for the fermentation, it is currently preferred that the bacteria and yeasts be capable of assimilating one or more of alcohols containing from 1 to 6 carbon atoms per molecule, for example methanol, ethanol, 1-propanol, 1-hexanol, and the like, as the source of carbon and energy in the growth or propagation of the microorganisms.

Suitable yeasts include species from the genera Candida, Hansenula, Torulopsis, Saccharomyces, Pichia, Debaryomyces, Lipomyces, Cryptococcus, Nematospora, and Brettanomyces. The preferred genera include Candida, Hansenula, Torulopsis, Pichia, and Saccharomyces. Examples of suitable species include:

| | |
|---|---|
| Candida boidinii | Candida mycoderma |
| Candida utilis | Candida stellatoidea |
| Candida robusta | Candida claussenii |
| Candida rugosa | Brettanomyces petrophilium |
| Hansenula minuta | Hansenula saturnus |
| Hansenula californica | Hansenula mrakii |
| Hansenula silvicola | Hansenula polymorpha |
| Hansenula wickerhamii | Hansenula capsulata |
| Hansenula glucozyma | Hansenula henricii |
| Hansenula nonfermentans | Hansenula philodendra |
| Torulopsis candida | Torulopsis bolmii |
| Torulopsis versatilis | Torulopsis glabrata |
| Torulopsis molishiana | Torulopsis numodendra |
| Torulopsis nitratophila | Torulopsis pinus |
| Pichia farinosa | Pichia polymorpha |
| Pichia membranaefaciens | Pichia pinus |
| Pichia pastoris | Pichia trehalophila |
| Saccharomyces cerevisiae | Saccharomyces fragilis |
| Saccharomyces rosei | Saccharomyces acidifaciens |
| Saccharomyces elegans | Saccharomyces rouxii |
| Saccharomyces lactis | Saccharomyces fractum |

The above described yeasts can be grown in a batch or continuous fermentation process in the presence of oxygen, an alcohol, and an assimilable source of nitrogen. Various types of fermentation processes known in the art can be utilized. For example, a foam fermenter such as is described in U.S. 3,982,998 can be used.

Suitable bacteria include species from the genera Bacillus, Mycobacterium, Actinomyces, Nocardia, Pseudomonas, Methanomonas, Protaminobacter, Methylococcus, Arthrobacter, Methylomonas, Brevibacterium, Acetobacter, Micrococcus, Rhodopseudomonas, Corynebacterium, Rhodopseudomonas, Microbacterium, Achromobacter, Methylobacter Methylosinus, and Methylocystis. Preferred genera include Bacillus, Pseudomonas, Protaminobacter, Micrococcus, Arthrobacter and Corynebacterium.

Examples of suitable species include:

| | |
|---|---|
| Bacillus subtilis | Bacillus cereus |
| Bacillus aureus | Bacillus acidi |
| Bacillus urici | Bacillus coagulans |
| Bacillus mycoides | Bacillus circulans |
| Bacillus megaterium | Bacillus licheniformis |
| Pseudomonas methanolica | Pseudomonas ligustri |
| Pseudomonas orvilla | Pseudomonas methanica |
| Pseudomonas fluorescens | Pseudomonas aeruginosa |
| Pseudomonas oleovorans | Pseudomonas putida |
| Pseudomonas boreopolis | Pseudomonas pyocyanea |
| Pseudomonas methylphilus | Pseudomonas brevis |
| Pseudomonas acidovorans | Pseudomonas methanoloxidans |
| Pseudomonas aerogenes | Protaminobacter ruber |
| Corynebacterium simplex | Corynebacterium hydrocarbooxydans |
| Corynebacterium alkanum | Corynebacterium oleophilus |
| Corynebacterium hydrocarboclastus | Corynebacterium glutamicum |
| Corynebacterium viscosus | Corynebacterium dioxydans |
| Cornyebacterium alkanum | Micrococcus cerificans |
| Micrococcus rhodius | Arthrobacter rufescens |
| Arthrobacter parafficum | Arthrobacter simplex |
| Arthrobacter citreus | Methanomonas methanica |
| Methanomonas methanooxidans | Methylomonas agile |
| Methylomonas albus | Methylomonas rubrum |
| Methylomonas methanolica | Mycobaterium rhodochrous |
| Mycobacterium phlei | Mycobacyerium brevicale |
| Nocardia salmonicolor | Nocardia minimum |
| Nocardia corallina | Nocardia butanica |
| Rhodopseudomonas capsulatus | Microbacterium ammoniaphilum |
| Archromobacter coagulans | Brevibacterium butanicum |
| Brevibacterium roseum | Brevibacterium flavum |

_Brevibacterium lactofermentum_     _Brevibacterium paraffinolyticum_

_Brevibacterium ketoglutamicum_     _Brevibacterium insectiphilium_

Oxygen can be supplied to the fermentation process in the form of air or oxygen enriched air. The source of nitrogen for the fermentation can be any organic or inorganic nitrogen-containing compound which is capable of releasing nitrogen in a form suitable for metabolic utilization by the growing organism. Suitable organic nitrogen compounds include, for example, proteins, amino acids, urea, and the like. Suitable inorganic nitrogen sources include ammonia, ammonium hydroxide, ammonium nitrate, and the like. The presently preferred nitrogen sources for the present invention are ammonia and ammonium hydroxide because these compounds can be easily removed during the SCP production process and do not increase the salt content of the SCP product. The amount of $NH_4OH$ added will depend upon the pH desired for the reaction mixture. Without any added $NH_4OH$ the pH will generally be about 2, for a mineral nutrient medium of the type described below. Preferably for the utilization of yeasts in the fermentation process the pH is preferably in the range of approximately 3-5 and for the utilization of bacteria the pH should preferably be in the range of approximately 6-7.5.

Sufficient water is maintained in the fermentation means so as to provide for the particular requirements of the microorganism employed. Minerals, growth factors, vitamins, and the like generally are added in amounts which vary according to the particular requirements of the microorganisms and are generally known to those skilled in the art or are readily determined by those so skilled. A typical example of a suitable mineral nutrient medium is as follows:

| Component | Amount |
|---|---|
| $H_3PO_4$ (75%) | 15.86 ml |
| $K_2SO_4$ | 9.53 g |
| $MgSO_4 \cdot 7H_2O$ | 7.8 g |
| $CaCl_2 \cdot 2H_2O$ | 0.6 g |
| KOH (85%) | 2.6 g |
| Trace Mineral Solution | 5.2 ml |
| Tap Water | 1 liter |

The trace mineral solution as listed in the above recipe is as given below;

| Component | Amount |
|---|---|
| $FeSO_4 \cdot 7H_2O$ | 65 g |
| $ZnSO_4 \cdot 7H_2O$ | 20 g |
| $MnSO_4 \cdot H_2O$ | 3 g |
| $CuSO_4 \cdot H_2O$ | 6 g |
| $H_2SO_4$ (conc) | 5 ml |

Deionized water to make 1 liter of solution

The growth of the microorganism is sensitive to the operating temperature of the fermenter and each particular species of microorganism has an optimum temperature for growth. Exemplary fermentation temperatures are in the range of about 20°C to about 65°C. The temperature selected will generally depend upon the microorganism employed in the process since each species will have a somewhat different temperature/growth rate relationship.

Fermentation pressures are generally within the range of about 0.1 to about 100 atmospheres, more usually about 1 to about 30 atmospheres, and more preferably about 1 to about 5 atmospheres since the higher pressures mean a greater level of dissolved oxygen in the aqueous medium and usually higher productivities.

The fermenter effluent can be utilized directly in the nucleic acid reduction step. Although less preferred because an extra and expensive step is added to the process, the fermenter effluent can also be centrifuged or filtered or spray-dried to yield the SCP cells and the cells can be resuspended in a suitable liquid, e.g., water, before the ammonium hydroxide treatment or the SCP cells can be mixed directly with aqueous ammonium hydroxide.

## II. NUCLEIC ACID REMOVAL

According to the process of the instant invention, a fluid containing cells of at least one species of microorganism, which fluid is fermenter effluent in a preferred embodiment, is treated with ammonium hydroxide at a pH in the range of about 7 to about 10 at a temperature broadly in the range of about 65°C to about 99°C for a time effective to reduce the nucleic acid content to below a desired level without excessive protein loss.

For yeast cells, a preferred pH range for the reduction of nucleic acid content to a suitable level is in the range of about 8.5 to about 10.

In _Pichia_ a preferred range for reduction of nucleic acid levels to a suitable level is about 9.25 to about 10 for minimal loss of protein. For _Hansenula_ a preferred range is about 8.8 to about 9.6. And for bacteria a presently contemplated preferred range is about 7 to about 9.0 for minimal loss of protein.

In all cases, however, it is expected that treatment of single cell protein in the pH range of about 7 to about 10 will at least reduce the level of nucleic acid content of the single cell protein without causing substantial loss of protein. pH values above about 10 can result in an excessive loss in protein content and, at least in certain instances, in lysinolanine formation.

The ammonium hydroxide can be added as a concentrated aqueous solution or can be formed in situ by adding ammonia to the aqueous SCP mixture. Other bases can also be used to reduce nucleic acid content; however, $NH_4OH$ is preferred because $NH_4OH$ is easily removed during the drying process, and does not increase the salt content of the SCP product.

The nucleic acid removal is broadly carried out at a temperature of from about 65°C to about 99°C to produce SCP having a nucleic acid content compatible with the SCP constituting a major portion of the daily human protein requirement. At temperatures below about 65°C the nucleic acid removal is undesirably slow and at temperatures above about 99°C there is a larger than desired loss of protein. More preferably, the temperature range is between about 85°C and about 99°C to produce an SCP product having a nucleic acid content compatible with the SCP being used as the sole source of the daily human protein requirement without excessive protein loss. As is shown in the following examples, the reaction temperature chosen will be related to the pH. At pH levels near the lower end of the above described suitable range (near pH 7), higher temperatures within the specified temperature range can generally be used. At higher pH levels (near pH 10), lower temperatures within the specified temperature range can be used with satisfactory nucleic acid removal.

The time required for the nucleic acid reduction depends on the specific microorganism used and on the temperature and pH of the treatment. At lower temperatures and pH values, longer times will be required. At higher temperatures and pH values, shorter times will be suitable. Generally, the time can be selected over a broad range and will be from a few minutes to several hours. However, generally the time of treatment will be in the range of about 5 to about 60 minutes. The optimum time for the particular microorganism and temperature can be readily determined by a few well chosen experiments.

At the conclusion of the ammonium hydroxide treatment, the mixture is separated by such methods as centrifugation or filtration to recover the SCP with a decreased nucleic acid content. The moist SCP product is then preferably spray dried to evaporate the remaining water and $NH_4OH$ to produce a dried SCP product in which the salt content is not increased. The supernatant from the separation step contains the nucleic acids removed from the SCP. These nucleic acids can be recovered by techniques known in the art such as acidification or ultrafiltration.

The recovered nucleic acids can be treated by an enzymatic hydrolysis as is well known in the art to recover mononucleotides which are useful, for example, as flavor enhancers.

### III. EXAMPLES

As required, detailed embodiments of the present invention are disclosed herein, however, it is to be understood that the disclosed embodiments are merely exemplary of the invention which may be embodied in various forms. Therefore, specific details disclosed herein are not to be interpreted as limiting but merely as a basis for the claims and as a representative basis for teaching one skilled in the art to variously employ the present invention in any appropriate detailed system.

The following assay procedures were used in the examples.

Nucleic Acids. The total nucleic acids were extracted from the samples with 0.5 N (normal) aqueous perchloric acid at 70°C for 20 minutes. The nucleic acid content in the extract was determined spectrophotometrically at 260 nm (nanometers).

Protein. Protein was extracted from the sample with 5 ml (milliliters) of 0.8 N sodium hydroxide at 100°C for 5 minutes. The extract was cooled to room temperature and 200 μl (microliters) of 18.75 weight % $CuSO_4.5H_2O$ was added. The mixture was allowed to stand at room temperature (about 25°C) for 30 minutes and the protein content was measured by the absorbance of the protein-copper complex at 540 nm using bovine albumin serum as a standard.

Carbohydrate. For total carbohydrate measurement, the samples were extracted with 1 N HCl for 30 minutes at 100°C. The hydrolysates were cooled to room temperature (about 25°C) and neutralized with NaOH.

The total reducing sugars were then assayed by Nelson's test (J. M. Clark, Jr., "Experimental Biochemistry", W. H. Freeman and Co., San Francisco, Calif., 1963, pp. 12-14).

Lysinoalanine. The lysinoalanine levels in the samples were determined by the method described by Sakamoto, et al. (M. Sakamoto, F. Nakayama, and K. I. Kajiyama; M. Friedman, Ed., "Protein Crosslinking", Vol. 1, Plenum Press, N.Y. 1976, pp. 687-712.)

## EXAMPLE I

In a continuous aerobic fermentation process, methanol and an aqueous mineral salts medium in a volume ratio of 40 to 60, respectively, were fed individually to a fermenter inoculated with the yeast species Pichia pastoris Culture 21-1 deposited with the Northern Utilization and Research Division, Agricultural Research Service, U.S. Department of Agriculture, Peoria, Illinois, and assigned number NRRL Y-11430. The fermenter was a 1500-liter foam-filled fermenter with a liquid volume of about 610 liters, with automatic pH, temperature, and level control. Agitation was provided by a turbine (750 rpm) at the bottom of a draft tube. The aeration rate was about 4 volumes of air (at about 38 psig and about 25°C) per volume of ferment in the fermenter per minute. Anhydrous ammonia was added at such a rate as to maintain the pH of the fermentation mixture at about 3.5.

The aqueous mineral salts medium was prepared by mixing, with each liter of tap water, 15.86 ml 75 percent $H_3PO_4$, 9.53 g $K_2SO_4$, 7.8 g $MgSO_4 \cdot 7H_2O$, 0.6 g $CaCl_2 \cdot 2H_2O$, 2.6 g 85 percent KOH, and 5.2 ml of a trace mineral solution. Biotin was fed separately via the methanol stream at a rate of 1 ml per liter of methanol.

The trace mineral solution was prepared by mixing, for each liter of solution, 65 g $FeSO_4 \cdot 7H_2O$, 20 g $ZnSO_4 \cdot 7H_2O$, 3.0 g $MnSO_4 \cdot 7H_2O$, 3.0 g $MnSO_4 \cdot H_2O$, 6.0 g $CuSO_4 \cdot 5H_2O$, 5.0 ml conc. $H_2SO_4$, and sufficient deionized water to make 1 liter of solution.

The aqueous mineral salts medium was fed at a rate of 27-30 liters per hour and the methanol at a rate of 18-20 liters per hour.

The fermentation was conducted at 30°C and about 38 psig pressure, with a retention time of 13.5-12.2 hours.

For analytical purposes yeast cells were separated from the fermentation effluent by centrifugation, washed by suspension in water and recentrifugation, dried overnight at 100°C, and weighed. On a dried basis, the yeast cells were produced in a yield of about 40 g per 100 g of methanol feed, the cell density being about 125 g of cells (dry weight) per liter of effluent.

### EXAMPLE II

A run was carried out to demonstrate the process of the present invention for the reduction of the nucleic acid content of a single cell protein. A mixture of 5 pints of concentrated ammonium hydroxide (28.4 weight % ammonia) and 50 gallons of the fermenter effluent from the aerobic fermentation of Pichia pastoris NRRL Y-11430 described in Example I was heated at 90°C for 20 minutes. The pH of the mixture was about 9.5. At the conclusion of the reaction time period, about 50 gallons of water was added and the resulting mixture was centrifuged and washed. The solid product was spray dried on an Anhydro Spray Dryer, Type III-A. The solid and the supernatant were assayed for protein and nucleic acid content. The results are presented in Table I along with results of a control assay of untreated SCP isolated from fermenter effluent by spray drying. The results are expressed on the basis of 100 g of starting SCP.

### TABLE I

| Sample | Fraction | Dry Weight, g | Protein, g | Nucleic Acids, g |
|---|---|---|---|---|
| Control | Solid | 100 | 58.3 | 6.2 |
| $NH_4OH$ | Solid | 86.1 | 52.4 | 1.05 |
| | Supernatant | 14 | N.D.[a] | N.D.[a] |

(a) N.D. = not determined.

The results in Table I demonstrate the process of the present invention for the reduction of nucleic acids in SCP. The solid product contains about 1.2 weight % nucleic acids (based on the dry weight of solid SCP product), a significant reduction from the 6.2 weight % nucleic acids in the control SCP sample, and less than the recommended 2% nucleic acid level which is preferably achieved if SCP is to constitute the sole source of human protein.

The amino acid content of the nucleic acid reduced SCP was determined and is presented in Table II. The fatty acid content of the nucleic acid reduced SCP was also determined and is presented in Table III. The corresponding values for a SCP produced directly from the fermenter effluent from the aerobic fermentation of Pichia pastoris are included in Tables II and III for comparison.

## TABLE II

| Amino Acid | Direct Dried SCP, Wt. %[a] | NH₄OH Treated SCP, Wt. %[a] |
|---|---|---|
| Alanine | 3.05 | 3.30 |
| Valine | 3.88 | 4.64 |
| Glycine | 2.75 | 3.30 |
| Isoleucine | 3.26 | 3.81 |
| Leucine | 4.38 | 5.22 |
| Proline | 2.14 | 2.54 |
| Threonine | 3.33 | 3.15 |
| Serine | 2.89 | 3.38 |
| Aspartic Acid | 5.31 | 5.83 |
| Methionine | 0.90 | 0.83 |
| Phenylalanine | 2.48 | 3.07 |
| Glutamic Acid | 7.04 | 6.80 |
| Tyrosine | 1.54 | 1.96 |
| Lysine | 3.66 | 4.23 |
| Arginine | 2.58 | 3.90 |

(a) Weight % of dry weight

## TABLE III

| Fatty Acid | Direct Dried SCP, Wt. %[a] | NH₄OH Treated SCP, Wt. %[a] |
|---|---|---|
| Myristic Acid | 0.33 | 1.06 |
| Petadecanoic Acid | 0.15 | 0.67 |
| Palmitic Acid | 12.99 | 15.93 |
| Hexadecenoic Acid | 5.67 | 11.31 |
| Margaric Acid | 0.43 | 0.25 |
| Heptadecenoic Acid | 1.97 | 3.07 |

| Stearic Acid | 2.53 | 1.13 |
| Oleic Acid | 37.88 | 26.55 |
| Linoleic Acid | 29.85 | 32.84 |
| Linolenic Acid | 4.00 | 5.76 |
| Light Fatty Acids[(b)] | 0.47 | 0.50 |
| Heavy Fatty Acids[(c)] | 1.37 | 0.84 |

(a) Weight % of total fatty acids
(b) Less than $C_{14}$
(c) Higher than $C_{18}$

An examination of the nucleic acid reduced SCP showed that the product had less of the typical yeasty taste and odor than the control SCP. In contrast to the yellow color of the control SCP, the treated SCP was lighter and slightly tan in color.

## EXAMPLE III

The lysinoalanine content of the nucleic acid reduced SCP from Example II was determined and was found to be only about 0.001 weight % lysinoalanine with the percentage based on the total dry SCP weight. For comparison, a sample of fermenter effluent from the fermentation of *Pichia pastoris* NRRL Y-11430, which was essentially the same as the fermenter effluent used in Example II, was treated with NaOH at pH 12 and 90°C for 20 minutes. An assay of the recovered SCP for lysinoalanine showed that 0.84 weight % lysinoalanine was present based on the total dry SCP weight.

These results show that the ammonium hydroxide treatment of the present invention yields a SCP with a reduced nucleic acid content and with essentially no lysinoalanine. A base treatment at a higher pH gave a SCP product with an appreciable amount of lysinoalanine. Since lysinoalanine is known to be undesirable for human consumption because of a tendency to cause formation of kidney lesions, the process of the present invention has an advantage over nucleic acid reduction methods using conditions under which non naturally occurring amino acids such as lysinoalanine are formed.

## EXAMPLE IV

An experiment was carried out to demonstrate the changes in the SCP protein, nucleic acids, carbohydrates, and lipids content that result from the ammonium hydroxide treatment of the present invention.

0041650

Four samples of fermenter effluent from the aerobic fermentation of Pichia pastoris NRRL Y-11430 described in Example I were used. Sample 1 was a control sample which was kept in ice. Sample 2 was mixed with concentrated ammonium hydroxide ($NH_4OH$) in a ratio of $NH_4OH$/effluent of 14 ml/l (liter) (pH 9.25) and heated for one hour at 90°C. Sample 3 was mixed with $NH_4OH$ in a $NH_4OH$/effluent ratio of 28 ml/l (pH 9.91) and heated for 30 minutes at 85°C. Sample 4 was mixed with $NH_4OH$ in a ratio of $NH_4OH$/effluent of 28 ml/l (pH 9.91) and was heated for one hour at 80°C.

At the conclusion of the reaction time period, samples 2, 3, and 4 were cooled with ice and all 4 samples were centrifuged at 10,000 rpm for 20 minutes. The centrifuge solids were lyophilized. The centrifuge supernatants were heated in an oven at 110°C for about one hour to remove ammonia and were then lyophilized. The lyophilization products derived from the centrifuge solids and from the supernatants were analyzed. The results are presented in Table IV with the weights expressed in terms of 100 g of starting SCP solids. The inorganic salts content of the fermenter effluent was determined by the supernatant weight in sample I and was subtracted from each supernatant weight.

TABLE IV

| Sample | Fraction | Dry Wt. g. | Protein, g. | Nucleic Acids, g. | Carbohydrates, g | Lipids, [a] g | Other, [b] g |
|---|---|---|---|---|---|---|---|
| 1 | Solid | 100 | 59.3 | 7.8 | 14.3 | 1.3 | 17.3 |
|   | Supernatant | 0 [e] | | | | | |
| 2 | Solid | 81.9 | 54.0 | 1.8 | 12.4 | 4.6 | 9.1 |
|   | Supernatant | 17.7 [e] | 4.1 | 6.0 [c] | 1.9 | 0.02 | 5.7 |
| 3 | Solid | 83.5 | 55.7 | 1.8 | 10.6 | 4.7 | 10.7 |
|   | Supernatant | 17.2 [e] | 4.6 | 6.0 [c] | 3.9 | 0.02 | 2.9 |
| 4 | Solid | 82.3 | 52.9 | 1.7 | 12.2 | 4.77 | 10.7 |
|   | Supernatant | 17.8 [e] | 4.6 | 6.1 [c] | 2.1 | 0.02 | 4.9 |

(a) Assayed by the methanol-chloroform extraction procedure.

(b) Other materials - unidentified. Values by difference.

(c) Calculated by difference.

(e) Corrected for salt content.

The results in Table IV show that three different ammonium hydroxide treatments (2, 3, and 4) reduce the nucleic acid content significantly while removing only a small portion of the protein. The nucleic acid contents in the solid products from treatments 2, 3, and 4 were about 2.1-2.2% (dry weight) and are considerably below the 7.8 weight % nucleic acids in control sample 1.

## EXAMPLE V

The influence of pH on the removal of nucleic acids from SCP by treatment with ammonium hydroxide was examined. Several samples of the fermenter effluent from the fermentation of Pichia pastoris NRRL Y-11430 described in Example I were treated at 80°C for 1 hour with ammonium hydroxide at various pH levels. At the conclusion of the ammonium hydroxide treatment, the product mixtures were cooled, centrifuged, and washed. The solids from the centrifugations were analyzed for nucleic acid content and the results are presented in Table V.

TABLE V

| Sample No. | $NH_4OH$ Conc. | pH | Nucleic Acid Remaining in SCP, % [a] |
|---|---|---|---|
| 1 | 0.2 N | 9.25 | 33.5 |
| 2 | 0.4 N | 9.91 | 16.8 |
| 3 | 0.6 | 10.01 | 14.2 |
| 4 | 0.8 | 10.12 | 13.2 |
| 5 | 1.0 | 10.20 | 11.8 |
| 6 | 2.0 | 10.45 | 8.1 |
| 7 | none [b] | 3.6 | 81.7 |

(a) % of amount of nucleic acids present in original SCP.

(b) Water was used. The temperature for this control run was 90°C.

The results described in Table V show that $NH_4OH$ treatments at pH levels from 9.25 to 10.45 remove significant quantities of nucleic acids from SCP with the percentage of removal increasing with higher pH values. Sample number 7 is a control run using only water instead of $NH_4OH$ and less than 20% of the nucleic acids were removed.

In order to determine the influence of the various pH levels on the protein content of the treated SCP, several more samples of the fermenter effluent from the fermentation of Pichia pastoris NRRL Y-11430 described in Example I were treated at various pH levels at 90°C for 40 minutes. The product mixtures were cooled, centrifuged, and washed. The solids from the centrifugation were assayed for protein. The pH levels and protein contents are shown in Table VI.

TABLE VI

| Sample No. | pH | Protein In Centrifuge Solid % |
|---|---|---|
| 8 | 3.65 | 89.7 |
| 9 | 9.24 | 82.7 |
| 10 | 9.51 | 82.1 |
| 11 | 10.05 | 74.1 |
| 12 | 10.25 | 68.3 |
| 13 | 10.50 | 66.8 |

The results in Table VI show that the higher pH levels cause removal of larger quantities of protein. At pH values above about 10, the protein loss increases rapidly and becomes unacceptably large.

EXAMPLE VI

The influence of temperature on the removal of nucleic acids from SCP by treatment with ammonium hydroxide was examined. Samples of the fermenter effluent from the aerobic fermentation of Pichia pastoris NRRl Y-11430 described in Example I were treated at various temperatures with 0.2 N and 0.4 N $NH_4OH$ for 1 hour. The product mixtures were cooled, centrifuged, and washed. The solids from the centrifugations were analyzed for nucleic acid content. The results in Table VII are expressed as the nucleic acids present as a weight percentage of the original amount of nucleic acids present before the treatment. A control run (sample 19) at 90°C with no added $NH_4OH$ is included for comparison.

TABLE VII

| Sample No. | | Temp., °C | Nucleic Acid Remaining in Sample, Wt. %[a] | Nucleic Acid Wt. % of SCP Product[e] |
|---|---|---|---|---|
| 0.2 N NH$_4$OH[b] - | 1 | 0 | 101.5 | 8.1 |
| | 2 | 25 | 102.6 | 8.2 |
| | 3 | 40 | 93.8 | 7.5 |
| | 4 | 52 | 81.0 | 6.5 |
| | 5 | 70 | 44.3 | 3.5 |
| | 6 | 80 | 33.5 | 2.7 |
| | 7 | 85 | 15.4 | 1.2 |
| | 8 | 90 | 11.9 | 1.0 |
| | 9 | 95 | 11.2 | 0.9 |
| 0.4 N NH$_4$OH[c] - | 10 | 0 | 102.6 | 8.2 |
| | 11 | 25 | 96.1 | 7.7 |
| | 12 | 40 | 78.2 | 6.3 |
| | 13 | 52 | 64.7 | 5.2 |
| | 14 | 70 | 18.2 | 1.5 |
| | 15 | 80 | 16.8 | 1.3 |
| | 16 | 85 | 9.4 | 0.8 |
| | 17 | 90 | 9.0 | 0.7 |
| | 18 | 95 | 8.4 | 0.7 |
| No NH$_4$OH[d] - | 19 | 90 | 71.9 | - |

(a) Weight % nucleic acids present as a % of the nucleic acid content before treatment.

(b) The pH values at the start of the treatment at 0.2 N were in the range of 9.3 to 9.5.

(c) The pH values at the start of the treatments at 0.5 N were in the range of 9.8 to 9.9.

(d) Initial pH = 3.6.

(e) Calculated assuming an untreated value of 8% nucleic con-

tent by weight.

The results shown in Table VII show that temperatures below about 65°C are relatively inefficient for the removal of nucleic acids from SCP. Thus the temperatures below about 65°C did not reduce the nucleic acid content of the SCP to the point at which the SCP can constitute as much as 50% of the human daily protein requirement. At about 65°C and above, however, the SCP has a nucleic acid content such that the SCP can constitute as much as 50% of the human daily protein requirement. Inspection of the data also shows that at least above about 85°C, the SCP has a nucleic acid content compatible with the SCP providing the entire daily human requirement of protein.

The results of Table VII also show that generally treatment with 0.4 N $NH_4OH$ (at a higher pH) is more effective in reducing nucleic acid content than treatment with 0.2 N $NH_4OH$. Generally, so long as the pH is in the stated preferred ranges, it is expected that temperature treatments of between about 65°C and 99°C will reduce nucleic acid content to a level compatible with SCP contributing as much as 50% of the daily human protein requirement; and temperature treatments between about 85°C and about 99°C will produce SCP products having a nucleic acid content compatible with the SCP being the sole source of the daily human protein requirement.

The results of Table VII also show that a control run at 90° with only water and no $NH_4OH$ removed less than 30% of the nucleic acids.

In other experiments involving $NH_4OH$ treatments at about pH 9.5 for 40 minutes, it was shown that at 98°C, about 30% of the SCP protein was removed while at 95°C, about 27% of the protein was removed. The amount of protein removed during treatment at 90°C was only about 18%. This indicates that about 99°C is about the upper limit for nucleic acid removal without excessive protein loss and that protein loss becomes increasingly large above about 90°C.

## EXAMPLE VII

A study was made of the influence of time on the removal of nucleic acids from SCP by treatment with ammonium hydroxide. A series of 10 ml samples of the fermenter effluent from the aerobic fermentation of Pichia pastoris NRRL Y-11430 described in Example I was adjusted to 0.2 N with ammonium hydroxide (pH 9.3) and held at 90°C. Another series of 10 ml samples of the same fermenter effluent was adjusted to 0.4 N with ammonium hydroxide (pH 9.7) and was held at 85°C. Samples were taken from each series at intervals and analyzed for nucleic acid content. The results in Table VIII are expressed as the amounts of nucleic acids present as a percentage of the original nucleic acid content.

TABLE VIII

| | 0.2 N NH$_4$OH at 90°C | | | 0.4 N NH$_4$OH at 85°C | |
|---|---|---|---|---|---|
| Sample No. | Time, Min. | Nucleic Acids, Wt. %[a] | Sample No. | Time, Min. | Nucleic Acids, Wt. %[a] |
| 1 | 0 | 93.4 | 9 | 0 | 92.3 |
| 2 | 10 | 41.8 | 10 | 10 | 27.1 |
| 3 | 20 | 27.0 | 11 | 20 | 17.0 |
| 4 | 30 | 22.6 | 12 | 30 | 14.1 |
| 5 | 40 | 19.8 | 13 | 40 | 11.4 |
| 6 | 50 | 16.9 | 14 | 50 | 10.5 |
| 7 | 60 | 13.3 | 15 | 60 | 10.3 |
| 8 | 120 | 11.3 | 16 | 120 | 9.5 |

(a) Weight % nucleic acids remaining as a % of the nucleic acid content before treatment.

The results in Table VIII show that nucleic acid removal is faster at 85°C with 0.4 N NH$_4$OH than at 90°C with 0.2 N NH$_4$OH. Nucleic acid removal is rapid up to about 30 minutes and slows between 30 and 60 minutes. Little change was noted after 60 minutes.

EXAMPLE VIII

An ammonium hydroxide treatment according to the process of this invention was carried out on the SCP from the aerobic fermentation of Hansenula polymorpha. The Hansenula polymorpha was grown in a continuous aerobic fermentation in a 4-liter reactor at 40°C. Methanol was used as the source of carbon and energy by the growing microorganism and the aqueous mineral salts medium was the same as described in Example I. The fermentation was carried out at a cell density of 70-80 g (dry)/liter of broth.

Two series of 4.5 ml samples of the fermenter effluent from Hansenula polymorpha were mixed with 0.25 ml of water and 0.25 ml of 4 N NH$_4$OH to yield 5 ml samples with 0.2 N NH$_4$OH with pH values of 8.8 to 8.9. Two other series of 4.5 ml samples of the same fermenter effluent were mixed with 0.5 ml of 4 N NH$_4$OH to yield 5 ml samples containing 0.4 N

$NH_4OH$ and pH values of 9.4 to 9.6. These series were treated at various temperatures from 80 to 90°C and samples were taken intervallically from each series for analysis of nucleic acid content. The results are shown in Table IX.

TABLE IX

| 0.2 N $NH_4OH$ at 90°C | | 0.4 N $NH_4OH$ at 85°C | |
|---|---|---|---|
| Time, Min. | Nucleic Acids, Wt. %[a] | Time, Min. | Nucleic Acids, Wt. %[a] |
| 0 | 94.6 | 0 | 93.2 |
| 10 | 28.7 | 10 | 16.3 |
| 20 | 19.6 | 20 | 12.2 |
| 30 | 16.4 | 30 | 9.3 |
| 40 | 13.5 | 40 | 8.2 |
| 50 | 12.4 | 50 | 8.1 |
| 60 | 11.1 | 60 | 7.2 |
| 120 | 8.1 | 120 | 6.8 |

| 0.2 N $NH_4OH$ at 85°C | | 0.4 N $NH_4OH$ at 80°C | |
|---|---|---|---|
| Time, Min. | Nucleic Acids, Wt. %[a] | Time, Min. | Nucleic Acids, Wt. %[a] |
| 0 | 100 | 0 | 96.6 |
| 20 | 30.5 | 20 | 15.2 |
| 40 | 22.1 | 40 | 11.6 |
| 60 | 17.6 | 60 | 8.9 |
| 80 | 14.7 | 80 | 7.9 |
| 100 | 13.7 | 100 | 7.1 |
| 120 | 13.1 | 120 | 6.6 |

(a) Weight % nucleic acids remaining as a % of the nucleic acid content before treatment.

The results presented in Table IX demonstrate the effectiveness of ammonium hydroxide treatment of the present invention for the removal of a substantial portion of the nucleic acids from SCP from Hansenula polymorpha.

## EXAMPLE IX

The removal of nucleic acids from bacterial SCP by treatment with ammonium hydroxide illustrates the difference in ease of removal of nucleic acids between bacteria and yeasts. A thermophilic mixed culture of bacteria designated as mixed strain HTB-53 and deposited with the Northern Utilization and Research Division, U.S. Department of Agriculture, Peoria, Illinois. NRRL B-8158 was grown in a continuous aerobic fermentation in a 4-liter fermenter at 55°C. The pH was maintained at about 6.3 by the addition of ammonium hydroxide solution and methanol was used as the source of carbon and energy by the growing microorganism. The aqueous mineral salts solution employed is listed below:

### Mineral Salts Medium

| Component | Amount |
|---|---|
| $H_3PO_4$ (85%) | 2 ml |
| KCl | 1 g |
| $MgSO_4 \cdot 7H_2O$ | 1.5 g |
| $CaCl_2 \cdot 2H_2O$ | 0.2 g |
| NaCl | 0.1 g |
| Trace Mineral Solution | 5 ml |
| Distilled Water | To make 1 liter |

The trace mineral solution was prepared as described below:

### Trace Mineral Solution

| Component | Amount |
|---|---|
| $CuSO_4 \cdot 5H_2O$ | 0.06 g |
| KI | 0.08 g |
| $FeCl_3 \cdot 6H_2O$ | 4.80 g |
| $MnSO_4 \cdot H_2O$ | 0.30 g |
| $Na_2MoO_4 \cdot H_2O$ | 0.20 g |
| $ZnSO_4 \cdot 7H_2O$ | 2.00 g |

0041650

| $H_3BO_3$ | 0.02 g |
| $H_2SO_4$ (conc) | 3 ml |
| Distilled Water | To make 1 liter |

Several 4.5 ml portions of the fermenter effluent were mixed with various amounts of water and 4 N $NH_4OH$ to yield 5 ml samples at various pH levels from 6.3 to 9.5. These samples were heated at 80°C for one hour. At the conclusion of the reaction time period, the samples were cooled, diluted with cold water, and centrifuged. The centrifuge solids were analyzed for nucleic acid content. The results are shown in Table X.

TABLE X

| Sample No. | pH | Nucleic Acids Remaining,[a] % |
|---|---|---|
| 1 | 6.3 | 51.1 |
| 2 | 7.1 | 26.2 |
| 3 | 8.3 | 19.1 |
| 4 | 8.7 | 14.9 |
| 5 | 9.0 | 11.7 |
| 6 | 9.1 | 9.6 |
| 7 | 9.3 | 10.6 |
| 8 | 9.5 | 6.3 |

(a) Nucleic acids remaining in the treated SCP as a % of the dry SCP weight.

The removal of substantial amounts of nucleic acids from bacterial SCP is shown in Table X to be accomplished at pH levels as low as 7.1. At pH values above about 9.0 little reduction of nucleic acid content is noted. A comparison of these results with the results in Example V using a yeast at the same conditions of time and temperature demonstrates that the yeast generally required a higher pH for efficient nucleic acid removal than the bacteria.

The process of my invention can be employed in producing a reduced nucleic acid content single cell protein product having a nucleic acid content in a range suitable for human consumption.

## IV.  OXIDIZING TREATMENT

In a further aspect, my invention comprises treating single cell protein product, preferably though not necessarily having a reduced nucleic acid content, with an oxidizing agent to produce a single cell protein product having improved color, odor, and flavor.

The oxidizing agent can be any suitable oxidizing agent effective to produce a single cell protein product having improved color, odor, and flavor.  Preferably, the oxidizing agent is a peroxide because of ready availability and efficacy though, of course, other oxidizing agents known in the art can also be used.  The peroxide can be any peroxide selected from the group consisting of hydrogen peroxide, sodium peroxide, potassium peroxide, magnesium peroxide, calcium peroxide, and the like.  For reasons of relative inexpense and reactivity and because the excess compound can be easily removed by drying from the SCP, the presently most preferred peroxide is hydrogen peroxide.

The treatment in accordance with the invention comprises contacting SCP with the oxidizing agent under conditions effective for improving the color, odor, and flavor of the SCP.  Any cellular product consisting of microbial cells can be so treated although preferably the cellular product consists of nucleic acid reduced protein as herein described.  The nucleic acid reduced protein as herein described can be contacted with the oxidizing agent at any time.  For example, the oxidizing agent can be added to an ammonium hydroxide SCP mixture at the end of the nucleic acid reduction step.  Alternatively, the nucleic acid reduced protein can be separated from the ammonium hydroxide-SCP mixture for example, by centrifugation, spray drying, or the like; and the separated nucleic acid reduced protein can then be contacted with an oxidizing agent of the invention.  Other times of contacting will be readily apparent to one skilled in the art and are also of course within the scope of this invention.

Preferably the contacting takes place with the SCP in aqueous suspension because the preferred oxidizing agents are water soluble and an aqueous suspension facilitates the reaction.  The aqueous suspension can contain any suitable amount of SCP.  Preferably the aqueous suspension will have from about 1 to about 30 grams of SCP per 100 ml of total suspension.  The amount of peroxide utilized can be any suitable amount effective to oxidize the nucleic acid reduced protein to produce an SCP product of improved color, taste, and odor.  Preferably the amount of peroxide will be in the range from about 0.01 to about 1 gram of peroxide per 100 ml of SCP aqueous suspension. Normally, peroxide levels toward the lower end of the above peroxide range can be used with lower levels of SCP. Likewise, higher peroxide levels can be used with higher amounts of SCP.

Generally as indicated the peroxide is added to a slurry or aqueous suspension of nucleic acid reduced SCP and during the time of addition the slurry or suspension can be at any temperature between about room temperature and the desired peroxide treatment temperature. The peroxide treatment temperature can be broadly from about 40°C to about 90°C and more preferably from about 50°C to about 90°C. The time of treatment at the peroxide treatment temperature can be any time effective to produce a nucleic acid reduced SCP having improved color, taste, and odor. Preferably, the time of treatment is between about 5 and about 60 minutes. Preferably the peroxide-SCP slurry is stirred, agitated or mixed to provide good mixing.

Following the peroxide treatment, the peroxide treated nucleic acid reduced protein can be separated from the slurry by centrifugation, filtration, spray drying or the like.

The resulting SCP product has a lighter color and improved odor and taste characteristics compared with SCP which has not been peroxide treated.

### EXAMPLE X

An invention run was carried out to demonstrate the peroxide treatment of nucleic acid reduced SCP to produce an improved SCP product. A sample of the fermenter effluent from an aerobic fermentation of Pichia pastoris NRRL Y-11430 similar to the fermentation described in Example I was treated with aqueous $NH_4OH$ at a pH of 9.5 and a temperature of 90°C for 40 minutes to remove a portion of the nucleic acids. The resulting mixture was centrifuged and 30 weight % aqueous $H_2O_2$ was added to the product fraction to bring the $H_2O_2$ concentration of the product fraction to a final concentration of 0.06 weight percent. The mixture was then heated at 60° C for 15 minutes, cooled, and dried in a spray drier. The resulting SCP powder was lighter in color, had less odor, and had less yeasty taste than SCP powder prepared without the hydrogen peroxide treatment.

C L A I M S

1.    A method of reducing the nucleic acid level in single cell protein comprising in combination:

producing a fluid containing cells by carrying out fermentation of at least one species of microorganism  under aqueous fermentation conditions and removing at least a portion of the thus produced effluent as said fluid containing cells;

heating said fluid containing cells with $NH_4OH$ in a pH range of about 7 to 10 at a temperature in the range of about 65°C to about 99°C;  and

separating said cells from said fluid.


2.    A method as in claim 1 characterized by further comprising :

drying the thus separated cells to remove water and $NH_4OH$ thereby resulting in a dried single cell protein product.


3.    A method as in claim 1 characterized in that said temperature is in the range of about 85 to about 90°C.


4.    A method as in claim 3 characterized in that said pH is in the range of about 8.5 to about 10.0; and said microorganism is selected from the group consisting of yeast and bacteria capable of fermentation under aqueous fermentation conditions.


5.    A method as in claim 4 characterized in that said yeast is a genus selected from the group consisting of Candida, Hansenula, Torulopsis, Saccharomyces, Pichia, Debaryomyces, Lipomyces, Cryptococcus, Nematospora, and Brettanomyces; or

wherein said yeast is a genus selected from the group consisting of Candida, Torulopsis, Hansenula, and Saccharomyces;  or

-2-

wherein said genus is selected from the group consisting
of Pichia and Hansenula;  or

wherein said microorganism is one or more species selected
from the following group:

| | |
|---|---|
| Candida boidinii | Candida mycoderma |
| Candida utilis | Candida stellatoidea |
| Candida robusta | Candida claussenii |
| Candida rugosa | Brettanomyces petrophilium |
| Hansenula minuta | Hansenula saturnus |
| Hansenula californica | Hansenula mrakii |
| Hansenula silvicola | Hansenula polymorpha |
| Hansenula wickerhamii | Hansenula capsulata |
| Hansenula glucozyma | Hansenula henricii |
| Hansenula nonfermentans | Hansenula philodendra |
| Torulopsis candida | Torulopsis bolmii |
| Torulopsis versatilis | Torulopsis glabrata |
| -Torulopsis molishiana | Torulopsis numodendra |
| Torulopsis nitratophila | Torulopsis pinus |
| Pichia farinosa | Pichia polymorpha |
| Pichia membranaefaciens | Pichia pinus |
| Pichia pastoris | Pichia trehalophila |
| Saccharomyces cerevisiae | Saccharomyces fragilis |
| Saccharomyces rosei | Saccharomyces acidifaciens |
| Saccharomyces elegans | Saccharomyces rouxii |
| Saccharomyces lactis | Saccharomyces fractum ; or |

wherein said microorganism is one or more species selected
from the group consisting of:

| | |
|---|---|
| Hansenula minuta | Hansenula saturnus |
| Hansenula californica | Hansenula mrakii |
| Hansenula silvicola | Hansenula polymorpha |
| Hansenula wickerhamii | Hansenula capsulata |
| Hansenula glucozyma | Hansenula henricii |
| Hansenula nonfermentans | Hansenula philodendra |
| Pichia farinosa | Pichia polymorpha |

Pichia membranaefaciens     Pichia pinus

Pichia pastoris     Pichia trebalophila ; or

wherein said microorganism is Pichia pastoris; or

wherein said at least one species of microorganism is Pichia pastoris; and

said pH is in the range of about 9.25 to about 10.0; or

wherein said at least one species of microorganism is Pichia pastoris NRRL Y-11430; and

said pH is in the range of about 9.25 to about 10.0; or

wherein said fermentation is carried out at a pH in the range of about 3 to about 5.

6. A method as in claim 2 characterized in that said at least one species of microorganism is Hansenula polymorpha; and

said pH is in the range of about 8.8 to about 9.6.

7. A method as in claim 3 characterized in that said microorganism is a genus of bacteria selected from the group consisting of Bacillus, Mycobacterium, Actinomyces, Nocardia, Pseudomonas, Methanomonas, Protaminobacter, Methylococcus, Arthrobacter, Methylomonas, Brevibacterium, Acetobacter, Micrococcus, Rhodopseudomonas, Corynebacterium, Microbacterium, Achromobacter, Methylobacter, Methylosinus and Methylocystis.

8. A method as in claim 7 characterized in that said microorganism is selected from the group consisting of Bacillus, Pseudomonas, Protaminobacter, Micrococcus, Arthrobacter and Corynebacterium; or

wherein said microorganism is one or more species selected from the group:

| | |
|---|---|
| Bacillus subtilus | Bacillus cereus |
| Bacillus aureus | Bacillus acidi |
| Bacillus urici | Bacillus coagulans |
| Bacillus mycoides | Bacillus circulans |
| Bacillus megaterium | Bacillus licheniformis |
| Pseudomonas methanolica | Pseudomonas ligustri |
| Pseudomonas orvilla | Pseudomonas methanica |
| Pseudomonas fluorescens | Pseudomonas aeruginosa |
| Pseudomonas oleovorans | Pseudomonas putida |
| Pseudomonas boreopolis | Pseudomonas pyocyanea |
| Pseudomonas methylphilus | Pseudomonas brevis |
| Pseudomonas acidovorans | Pseudomonas methanoloxidans |
| Pseudomonas aerogenes | Protaminobacter ruber |
| Corynebacterium simplex | Corynebacterium hydrocarbooxydans |
| Corynebacterium alkanum | Corynebacterium oleophilus |
| Corynebacterium hydrocarboclastus | Corynebacterium glutamicum |
| Corynebacterium viscosus | Corynebacterium dioxydans |
| | |
| Cornyebacterium alkanum | Micrococcus cerificans |
| Micrococcus rhodius | Arthrobacter rufescens |
| Arthrobacter parafficum | Arthrobacter simplex |
| Arthrobacter citreus | Methanomonas methanica |
| Methanomonas methanooxidans | Methylomonas agile |
| Methylomonas albus | Methylomonas rubrum |
| Methylomonas methanolica | Mycobaterium rhodochrous |
| Mycobacterium phlei | Mycobacyerium brevicale |
| -Nocardia salmonicolor | Nocardia minimum |
| Nocardia corallina | Nocardia butanica |
| Rhodopseudomonas capsulatus | Microbacterium ammoniaphilum |
| Archromobacter coagulans | Brevibacterium butanicum |
| Brevibacterium roseum | Brevibacterium flavum |
| Brevibacterium lactofermentum | Brevibacterium paraffinolyticum |
| Brevibacterium ketoglutamicum | Brevibacterium insectiphilium ; or |

wherein said bacteria is a thermophilic mixed culture of bacteria designated as mixed strain HTB-53 and deposited as NRRL B-8158, in particular

wherein said pH is in the range from about 7 to about 9.0, in particular

wherein said fermentation is carried out in a pH range from about 6 to about 7.5 .

9.    A method for producing a single cell protein product characterized by oxydizing a cellular product consisting of microbial cells by contacting the cellular product with an oxydizing agent under oxidizing conditions; and separating out the thus oxidized cellular product.

10.    A method as in claim 9 characterized in that the cellular product consists of cells prepared according to any of claims 1, 4, 5 or 7 in the alternative;   or

wherein the oxidizing agent is selected from the group consisting of hydrogen peroxide, potassium peroxide, sodium peroxide, magnesium peroxide, and calcium peroxide;   or

wherein the oxidizing agent is hydrogen peroxide;   or

wherein the contacting of the cellular product with oxidizing agent takes place with the cellular product in aqueous suspension;   or

wherein the contacting of the cellular product with oxidizing agent takes place with the cellular product in an aqueous suspension comprising the range of about 1 to about 30 grams of cells per 100 milliliters of aqueous suspension; and

the oxidizing agent is hydrogen peroxide, present in an amount in the range of about 0.01 to about 1 gram per 100 milliliters of aqueous suspension, in particular

wherein the hydrogen peroxide is present in an amount comprising about 0.06 percent by weight of the aqueous suspension;   or wherein the contacting of the cellular product with oxidizing agent takes place in aqueous suspension;

said contacting in aqueous suspension is carried out at a temperature in the range of about 40°C to about 90°C; and

said contacting in aqueous suspension is carried out for a time in the range fo about 5 to about 60 minutes, in particular

wherein the temperature is in a range of about 50°C to about 90°C.